# EUROPÄISCHE PATENTSCHRIFT

(11) **EP 0 339 393 B1**
(45) Veröffentlichungstag und Bekanntmachung des Hinweises auf die Patenterteilung: **24.03.1993**
(21) Anmeldenummer: 89106692.0
(22) Anmeldetag: 14.04.1989
(51) Int. Cl.: C07C 381/00, C08F 12/00

(54) **Polymerisierbare Aryldiazosulfonate und Polymere enthaltend wiederkehrende Einheiten mit Aryldiazosulfonatgruppen**
Polymerisable aryl diazo sulfonates and polymers containing repeating units with aryl diazo sulfonate groups
Diazosulfonates d'aryle polymérisables et polymères contenant des unités périodiques portant des groupes aryldiazolsulfonates

(30) Priorität: 27.04.1988 DE 3814164
(43) Veröffentlichungstag der Anmeldung: 02.11.1989
(73) Patentinhaber: BAYER AG, 51368 Leverkusen (DE)
(72) Erfinder: Nuyken, Oskar, Prof. Dr., D-8000 München 81 (DE); Knepper, Thomas, D-6581 Schauren (DE); Voit, Brigitte, D-8580 Bayreuth (DE); Pask, Stephen David, Dr., D-4047 Dormagen (DE)

(56) Entgegenhaltungen:
- EP-A- 0 068 338
- GB-A- 877 402
- CHEMICAL ABSTRACTS, Band 83, Nr. 8, 25. August 1975, Seite 173, Zusammenfassung Nr. 61601g, Columbus, Ohio, US; A.P. DONYA et al.: "Synthesis of triazenes and azo dyes from aminostyrenes"

## Beschreibung

Die Erfindung betrifft polymerisierbare Gruppen enthaltende Aryldiazosulfonate, ein Verfahren zu ihrer Herstellung, die Verwendung der neuen Aryldiazosulfonate zur Herstellung von Polymerisaten und Polymere enthaltend wiederkehrende Einheiten mit Aryldiazosulfonatgruppen.

"Aryldiazosulfonate" im Sinne der Erfindung sind Verbindungen mit (pro Molekül) mindestens einer Sulfonatgruppe, die direkt an die Diazogruppe gebunden ist. Die üblichen Azofarbstoffe sind (abgesehen von fehlenden polymerisierbaren Gruppen) also - weil Diazogruppen und Sulfonatgruppen jeweils durch irgendwelche Struktureinheiten getrennt sind - keine Aryldiazosulfonate im Sinne der Erfindung.

"...sulfonat" im Sinne der Erfindung bedeutet -SO₃M, worin M für Wasserstoff, NH₄, NR′₄ (worin R′ = CH₃, C₂H₅, C₃H₇ oder C₄H₉), Al, Zn oder Cu, vorzugsweise aber Alkalimetall oder ein Äquivalent Erdalkalimetall steht.

Wir haben nun erstmalig Aryldiazosulfonate mit radikalisch polymerisierbaren ethylenisch ungesättigten Gruppen synthetisiert. Diese neuen Aryldiazosulfonate lassen sich zu Homo- und Copolymerisaten polymerisieren, von denen insbesondere die wasserlöslichen Polymerisate äußerst interessant sind. Durch Bestrahlung lassen sich wasserlösliche Polymere enthaltend wiederkehrende Einheiten mit Aryldiazosulfonatgruppen in wasserunlösliche Produkte umwandeln; es darf angenommen werden, daß durch die Bestrahlung die Diazogruppe gespalten oder abgespalten wird. Eventuell werden zunächst die entsprechenden Diazonium-Kationen gebildet, die unter Abspaltung von Stickstoff in Gegenwart von Wasser in das entsprechende Phenol übergehen. Die Wasserlöslichkeit der Polymerisate läßt sich durch Bestrahlung so weit herabsetzen, daß sich die unterschiedliche Wasserlöslichkeit bestrahlter und nicht bestrahlter Partien technisch sinnvoll nutzen läßt; die nicht bestrahlten Partien können beispielsweise mit Wasser abgewaschen werden, so daß auf diese Weise Bilder entstehen, z.B. auf hydrophilen Trägern wie Aluminium zur Herstellung von Druckplatten.

Gegenstand der Erfindung sind also Aryldiazosulfonate mit
(i) mindestens 1, vorzugsweise 1 bis 4, aromatischen C₆-C₁₀-Rest(en),
(ii) mindestens 1, vorzugsweise 1 bis 3, an einen aromatischen Rest gebundenen Diazosulfonatgruppe(n) und
(iii) mindestens 1, vorzugsweise 1 bis 2, an einen aromatischen C₆-C₁₀-Rest gebundenen radikalisch polymerisierbaren ethylenisch ungesättigten Gruppe(n).

Bevorzugte ungesättigte Gruppen (iii) enthalten 1 bis 2 polymerisierbare C=C-Doppelbindungen pro Gruppe.

Bevorzugte aromatische Reste (i) umfassen Phenylen und Naphthylen; bevorzugte ungesättigte Gruppen (iii) entsprechend der Formel

X-Rₙ- (I),

worin
- X: -C=CH₂, -CCH₃=CH₂, -CH=CHCH₃, -CCN=CH₂, -CH=CHCN, -CCl=CH₂, -CH=CHCl, -OCOCH=CH₂, -OCOCH=CHCH₃, -OCOCCH₃=CH₂, -COOCCH₃=CH₂, -COOCH=CHCH₃, -CH=CH-CH=CH₂, -O-CH=CH₂, -O-CCH₃=CH₂ oder -O-CH=CH-CH₃, vorzugsweise -CH=CH₂, -CCH₃=CH₂, -OCOCCH₃=CH₂ oder -OCOCH=CH₂,
- R: C₁-C₁₀-Alkylen, C₆-C₁₈-Arylen, C₁-C₁₀-Alkylenoxy, C₆-C₁₈-Arylenoxy, C₁-C₁₀-Alkylencarboxyamino, C₁-C₁₀-Alkylencarbamoyl, C₇-C₁₉-Arylencarbamoyl, C₁-C₁₀-Alkylenamino und C₆-C₁₈-Arylenamino und
- n: Null oder 1 bedeuten.

Besonders bevorzugte Aryldiazosulfonate sind solche, deren Diazosulfonatgruppen (ii) an die gleich aromatischen Reste (i) wie die polymerisierbaren Gruppen (iii) gebunden sind.

Die bevorzugten Aryldiazosulfonate sind solche der Formel 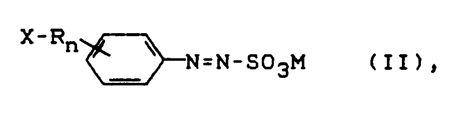
worin M die obengenannte Bedeutung besitzt und vorzugsweise für Kalium oder Natrium steht. In den bevorzugtesten Aryldiazosulfonaten bedeuten X -CH=CH₂ und n Null.

Die erfindungsgemäßen Aryldiazosulfonate lassen sich analog den Vorschriften aus "Methoden der Organischen Chemie" (Houben-Weyl), Bd. 10/3, Georg Thieme Verlag, Stuttgart 1965, bzw. DE-OS 31 25 104 aus polymerisierbaren Gruppen enthaltenden Arylaminen durch Diazotierung und anschließende Umsetzung mit Sulfit, vorzugsweise Alkali- oder Erdalkalisulfit, herstellen. Die Reaktionwird vorzugsweise bei Temperaturen von -20 bis 120, insbesondere von 0 bis 50, am besten von 0 bis 30, °C und bei einem pH-Wert von 4 bis 13, insbesondere von 4 bis 5 oder 8 bis 10, durchgeführt.

Die neuen Aryldiazosulfonate können zu Homo- und Copolymeren polymerisiert werden, die Aryldiazosulfonatgruppen in der Kette enthalten. Nach einer anderen Variante können solche Polymeren dadurch hergestellt werden, daß man zuerst polymerisierbare Gruppen enthaltende Arylamine polymerisiert und dann die Aminogruppen durch Diazotierung und Reaktion mit Sulfit - wie oben beschrieben - zu Diazosulfonatgruppen umsetzt.

Für die Copolymerisation mit den erfindungsgemäßen Aryldiazosulfonaten eignen sich radikalisch polymerisierbare Monomere, vorzugsweise solche mit 1 bis 2 ethylenisch ungesättigten polymerisierbaren Gruppen und mit 2 bis 12 C-Atomen, wie z.B. Styrol, Methylmethacrylat, Acrylnitril, Methylacrylat, Ethylacrylat, Acrylamid, Methacrylamid, Acrylsäure, Methylacrylsäure, Ethylacrylsäure, Butadien und Isopren sowie Gemische aus zwei oder mehreren dieser Monomeren. Styrol, Acrylnitril, Butadien und Methylmethacrylat sind bevorzugte Comonomere.

Sowohl die Homo- als auch die Copolymerisate können nach an sich bekannten Methoden in Lösung oder in Emulsion hergestellt werden. Die Molekulargewichte der Homo- und Copolymerisate können 10.000 bis 1.000.000, vorzugsweise 10.000 bis 100.000 betragen, gemessen durch Gelpermeationschromatographie an Polystyrolsäulen mit Tetrahydrofuran als Elutionsmittel, verglichen mit Polymethylmethacrylat als Standard; Porengrößen der Standarde: 10⁶, 10⁴, 10³ und 500 Ångström.

Aus den neuen Aryldiazosulfonaten erhältliche Copolymerisate können 1 bis 99, vorzugsweise 10 bis 50, Mol-% Aryldiazosulfonatgruppen enthaltende Einheiten und 99 bis 1, vorzugsweise 90 bis 50, Mol-% copolymerisierte Einheiten eines oder mehrerer anderer Monomerer enthalten.

Die erfindungsgemäßen Homo- und Copolymeren können durch ihren Gehalt an wiederkehrenden Einheiten mit Aryldiazosulfonatgruppen charakterisiert werden, wobei diese Aryldiazosulfonatgruppen
(i) mindestens 1, vorzugsweise 1 bis 4, aromatische(n) C₆-C₁₀-Rest(e) und
(ii) mindestens 1, vorzugsweise 1 bis 3, an einen aromatischen Rest gebundene Diazosulfonatgruppe(n) enhält.

Bevorzugte aromatische Reste sind Phenylen und Naphthylen.

Wünscht man wasserlösliche Copolymerisate, so hängt die erforderliche Mindestmenge der dazu notwendigen Aryldiazosulfonatgruppen von der Polarität der verwendeten Monomeren und vom Molekulargewicht des gewünschten Copolymerisats ab. Im allgemeinen werden Copolymerisate aus 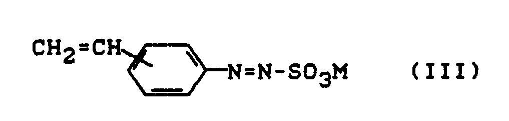
und Styrol ab 25 Mol-% III, Copolymerisate aus III und Methylmethacrylat ab 15 Mol-% III wasserlöslich.

Besonders bevorzugte wasserlösliche Copolymerisate bestehen zu mehr als 90, insbesondere zu 100, Gew.-% aus Einheiten mit Aryldiazosulfonatgruppen, Styrol und/oder Methylmethacrylat.

"Wasserlöslich" im Sinne der Erfindung bedeutet, daß bei einer Konzentration von mindestens 35 g Polymer pro Liter Wasser bei 45° C nicht mehr als 1 g Polymer unlöslich ist.

Die wasserlöslichen Homo- und Copolymerisate lassen sich problemlos aus wäßriger Lösung zu Filmen verarbeiten, wobei die Trocknung vorzugsweise bei erhöhter Temperatur und gegebenenfalls reduziertem Druck erfolgen kann. Die Filme können selbsttragend oder auf einen Träger aufgebracht sein.

Die wasserlöslichen Polymerisate mit wiederkehrenden Einheiten enthaltend Aryldiazosulfonatgruppen können durch energiereiche Strahlung, wie z.B. UV-Licht, Röntgen- und Elektronenstrahlen zu wasserunlöslichen Produkten umgesetzt werden. Die Geschwindigkeit der Reaktion hängt von Wellenlänge, Lichtstärke und gegebenenfalls Strahlerabstand vom Polymerisat ab. Für viele Fälle dürfte eine Quecksilber-Xenon-Hochdrucklampe geeignet sein. Ein Film einer Schichtdicke von 0,1 mm eines Copolymerisats aus 80 Mol-% Methylmethacrylat und 20 Mol-% III ist z.B. nach 10 Minuten Bestrahlung mit einer Quecksilber-Xenon-Hochdrucklampe (132 W, Abstand 30 cm) wasserunlöslich. Optimale Ergebnisse lassen sich durch Bestrahlung mit einer Wellenlänge entsprechend dem Absorptionsmaximum des Polymeren erreichen.

Die erfindungsgemäßen Polymerisate lassen sich als Bindemittel für Druckfarben und dekorative Überzugsmittel, als Photoresists für gedruckte Schaltungen, zur Herstellung von elektronischen Bausteinen und als Überzüge auf hydrophilen Trägern zur Herstellung von Druckplatten etc. durch gezielte Bestrahlung mit nachfolgender Entwicklung durch Abwaschen verwenden.

In den folgenden Beispielen beziehen sich die Prozentangaben jeweils aus das Gewicht.

### Beispiele

### Beispiel 1

### Synthese des 3-Vinylphenyldiazosulfonat-Natriumsalzes

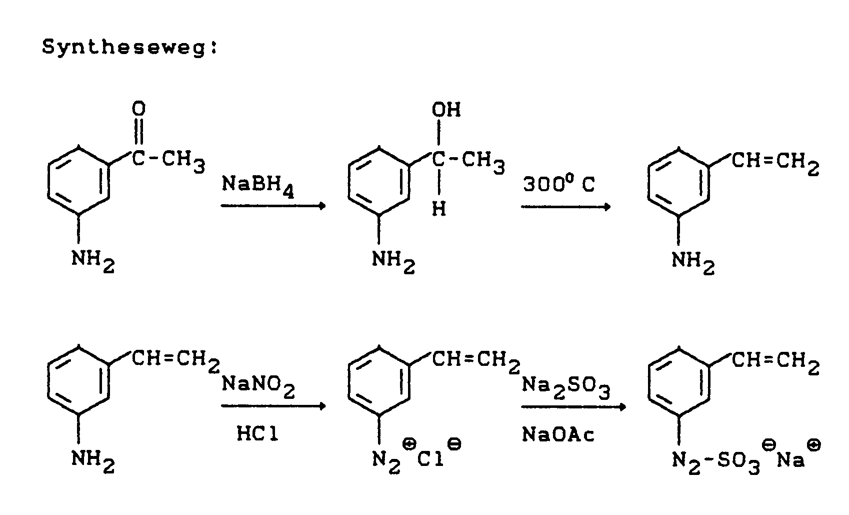

### Arbeitsvorschrift:

### 3-Aminostyrol

135 g (1 mol) 3-Aminoacetophenon werden in 1000 ml Isopropanol aufgeschlämmt und langsam mit 19 g (0,5 mol) Natriumborhydrid versetzt. Das Reaktionsgemisch wird 48 Stunden bei Raumtemperatur gerührt und dann unter Eiskühlung mit konzentrierter Salzsäure auf pH = 5 eingestellt. Das ausfallende Salz wird abgesaugt und die leicht rötliche Lösung am Rotationsverdampfer im Wasserstrahlvakuum eingeengt. Das gewünschte 1-(3-Aminophenyl)-ethanol kristallisiert über Nacht in der Kälte aus, wird abgenutscht und getrocknet.

Der Alkohol wird anschließend bei 300° C mit Hilfe eines Aluminiumoxidkatalysators in einem beheizten Rohr dehydratisiert. Das Produkt wird mit Wasser aufgenommen, durch Ausschütteln mit Ether aus der wäßrigen Phase extrahiert, die organische Phase über Natriumsulfat getrocknet und das Lösungsmittel abgezogen.
Ausbeute: 77,3 g.

### 3-Vinylphenyldiazosulfonat-Natriumsalz

12 g (0,1 mol) 3-Aminostyrol werden in 100 ml 10 %iger wäßriger Salzsäure auf -5° C abgekühlt. Dazu wird langsam eine vorgekühlte Lösung von 6,9 g (0,1 mol) Natriumnitrit in 50 ml Wasser zugetropft. Die Reaktionslösung wird noch 30 Minuten bei 0° C gerührt und dann schnell einer -5° C kalten Lösung von 25,2 g (0,2 mol) Na₂SO₃ und 33 g (0,4 mol) Natriumacetat in 150 ml Wasser zugegeben. Das Produkt fällt bei Eiskühlung aus und kann abgenutscht werden. Es wird mit wenig kaltem Wasser gewaschen und in Ethanol umkristallisiert. Man erhält 11,7 g gelbes Pulver.

### Beispiel 2

### Synthese des Benzylmethacrylat-3-diazosulfonat-Natriumsalzes

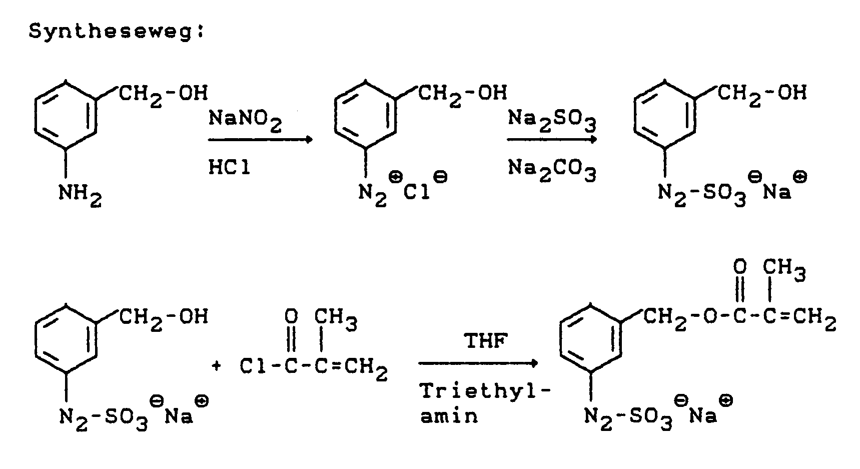

Der Aminobenzylalkohol wird analog dem 3-Aminostyrol (Beispiel 1) diazotiert und mit Natriumsulfit umgesetzt.

0,1 Mol des erhaltenen Benzylalkohol-3-diazosulfonat-Natriumsalzes werden in 500 ml THF gelöst und auf -10° C gekühlt. Dann werden 0,12 Mol Methacrylsäurechlorid zugegeben. Diesem Gemisch werden 0,12 Mol Triethylamin, gelöst in 150 ml THF, tropfenweise zugesetzt und anschließend wird noch 15 min bei -10° C weitergerührt. Anschließend wird die Mischung auf Raumtemperatur erwärmt und noch eine weitere Stunde gerührt. Das ausgefallene Produkt wird dann abgesaugt und aus Ethanol umkristallisiert.
Ausbeute: 14.3 g

### Anwendungsbeispiele 3-5

Copolymerisate aus 3-Vinylphenyl-diazosulfonat und Methylmethacrylat (MMA).

| Reaktionsansatz: | |
|---|---|
| 0,05 Mol | Methylmethacrylat + Vinylphenyldiazosulfonat-Na |
| 0,3 mMol | Azoisobutyronitril (= 0,05 g) bzw. |
| 0,6 mMol | Azoisobutyronitril (= 0,1 g) |
| 50 ml | Dioxan/Wasser (Volumenverhältnis 8:2), entgast. |

Die Polymerisation wird bei 70° C unter Schutzgas (N₂) durchgeführt. Reaktionsgefäß und Reaktionslösung werden durch Spülen mit Stickstoff von Sauerstoff befreit. Das Lösungsmittel und die Monomeren werden vorgelegt. Anschließend wird die Polymerisation durch Zugabe des Initiators gestartet. Die Reaktionszeiten liegen, je nach Azogehalt im Ansatz, zwischen 3 und 6 Stunden. Der Abbruch der Polymerisation erfolgt durch Eiskühlung und Zugabe von etwas Hydrochinon. Das Lösungsmittel und nicht umgesetztes MMA werden im Wasserstrahlvakuum am Rotationsverdampfer abgezogen, das erhaltene Produkt in Dioxan/Chloroform (Volumenverhältnis 4:1) (≧90 Mol-% MMA) bzw. in Chloroform/ Methanol (Volumenverhältnis 1:1) (<90 Mol-% MMA) gelöst und durch Eingießen dieser Lösung in den 10-fachen Überschuß, bezogen auf das Gewicht, Hexan gefällt; das Copolymerisat wird abfiltriert, kurz mit eiskaltem Wasser gewaschen und im Vakuum bis zur Gewichtskonstanz getrocknet.

**Tabelle 1**

| Beispiel | MMA-Gehalt d. Monomergemisches (Mol-%) | Reaktionszeit (Stunden) | Azogehalt im Polymer* (Gew.-%) | Molekulargewicht (GPC/THF) |
|---|---|---|---|---|
| 3 | 90 | 3 | 7 | 20.000 |
| 4 | 85 | 4 | 10 | 16.000 |
| 5 | 80 | 6 | 12 | 11.500 |

| | | | | |
|---|---|---|---|---|
| * bestimmt aus der UV-Absorption bei 291 nm und dem Absorptionskoeffizienten für 3-Methylphenyldiazosulfonat-Na. | | | | |

Das Copolymerisat aus Beispiel 3 war in Methanol/Wasser (1:1 Volumenteile) löslich, die Copolymerisate aus den Beispielen 4 und 5 waren wasserlöslich. Die Copolymerisate werden 10 gew.-%ig in diesen Lösungsmitteln bei 65° C gelöst; aus diesen Lösungen werden Filme einer Trockenfilmschichtdicke von 0,1 mm auf Glasträgern hergestellt. Die Filme werden bei 80° C im Vakuum (0,1 Torr) 3 Stunden getrocknet. Nach dem Trocknen sind die Filme - wie Kontrollversuche zeigen - in den genannten Lösungsmitteln noch löslich. Nach einer Bestrahlung mit einer Quecksilber-Xenon-Hochdrucklampe (132 Watt, Abstand 20 cm) sind die Filme in den genannten Lösungsmitteln unlöslich, so daß nur unbestrahlte Regionen abgewaschen werden konnten.

## Patentansprüche

1. Aryldiazosulfonate mit
(i) mindestens einem aromatischen C₆-C₁₀-Rest,
(ii) mindestens einer an einen aromatischen Rest gebundenen Diazosulfonatgruppe und
(iii) mindestens einer an einen aromatischen C₆-C₁₀-Rest gebundenen radikalisch polymerisierbaren ethylenisch ungesättigten Gruppe.

2. Aryldiazosulfonate nach Anspruch 1, deren aromatische Reste (i) Phenylen und/oder Naphthylen sind.

3. Aryldiazosulfonate nach Ansprüchen 1 und 2, deren ungesättigte Gruppe (ii) 1 bis 2 polymerisierbare C=C-Doppelbindungen enthalten.

4. Aryldiazosulfonate nach Ansprüchen 1 bis 3, deren ungesättigte Gruppen (iii) der Formel
X-Rₙ- (I)
entsprechen, worin
X -C=CH₂, -CCH₃=CH₂, -CH=CHCH₃, -CCN=CH₂, -CH=CHCN, -CCl=CH₂, -CH=CHCl, -OCOCH=CH₂, -OCOCH=CHCH₃, -OCOCCH₃=CH₂, -COOCCH₃=CH₂, -COOCH=CHCH₃, -CH=CH-CH=CH₂, -O-CH=CH₂, -O-CCH₃=CH₂ oder -O-CH=CH-CH₃,
R C₁-C₁₀-Alkylen, C₆-C₁₈-Arylen, C₁-C₁₀-Alkylenoxy, C₆-C₁₈-Arylenoxy, C₁-C₁₀-Alkylencarboxyamino, C₁-C₁₀-Alkylencarbamoyl, C₇-C₁₉-Arylencarbamoyl, C₁-C₁₀-Alkylenamino und C₆-C₁₈-Arylenamino und
n Null oder 1 bedeuten.

5. Aryldiazosulfonate nach Ansprüchen 1 bis 4, wobei die Diazosulfonatgruppen (ii) an die gleichen aromatischen Reste (i) wie die polymerisierbaren Gruppen (iii) gebunden sind.

6. Aryldiazosulfonate der Formel 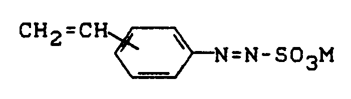 worin M für Wasserstoff, NH₄, NR′₄ (mit R′ = C₁-C₄-Alkyl), Al, Ni, Zn, Cu, Alkalimetall oder 1 Äquivalent Erdalkalimetall steht.

7. Verfahren zur Herstellung der Aryldiazosulfonate nach Ansprüchen 1 bis 6 durch Diazotierung von polymerisierbare Gruppen enthaltenden Arylaminen und anschließende Umsetzung mit Sulfit.

8. Verwendung der Aryldiazosulfonate nach Ansprüchen 1 bis 6 zur Herstellung von Polymerisaten.

9. Polymere enthaltend wiederkehrende Einheiten mit Aryldiazosulfonatgruppen, wobei diese Einheiten
(i) mindestens 1 aromatischen C₆-C₁₀-Rest und
(ii) mindestens 1 an einen aromatischen Rest gebundene Diazosulfonatgruppe enthalten.

10. Polymere nach Anspruch 9, wobei die aromatischen Reste (i) Phenylen und/oder Naphthylen sind.

11. Polymere nach Ansprüchen 9 oder 10, worin die Diazosulfonatgruppen-enthaltenden Einheiten von Aryldiazosulfonaten nach Ansprüchen 1 bis 6 abgeleitet sind.

## Claims

1. Aryldiazosulfonates containing
(i) at least one aromatic C₆₋₁₀ radical,
(ii) at least one diazosulfonate group attached to an aromatic radical and
(iii) at least one radical-polymerizable ethylenically unsaturated group attached to an aromatic C₆₋₁₀ radical.

2. Aryldiazosulfonates as claimed in claim 1 of which the aromatic radicals (i) are phenylene and/or naphthylene.

3. Aryldiazosulfonates as claimed in claims 1 and 2 of which the unsaturated groups (ii) contain 1 to 2 polymerizable C=C double bonds.

4. Aryldiazosulfonates as claimed in claims 1 to 3 of which the unsaturated groups (iii) correspond to the formula
X-Rₙ- (I)
in which
X represents-C=CH₂, -CCH₃=CH₂, -CH=CHCH₃, -CCN=CH₂, -CH=CHCN, -CCl=CH₂, -CH=CHCl, -OCOCH=CH₂, -OCOCH=CHCH₃, -OCOCCH₃=CH₂, -COOCCH₃=CH₂, -COOCH=CHCH₃, -CH=CH-CH=CH₂, -O-CH=CH₂, -O-CCH₃=CH₂ or -O-CH=CH-CH₃,
R represents C₁₋₁₀ alkylene, C₆₋₁₈ arylene, C₁₋₁₀ alkyleneoxy, C₆₋₁₈ aryleneoxy, C₁₋₁₀ alkylenecarboxyamino, C₁₋₁₀ alkylenecarbamoyl, C₇₋₁₉ arylenecarbamoyl, C₁₋₁₀ alkyleneamino and C₆₋₁₈ aryleneamino and
n is 0 or 1.

5. Aryldiazosulfonates as claimed in claims 1 to 4 in which the diazolesulfonate groups (ii) are attached to the same aromatic radicals (i) as the polymerizable groups (iii).

6. Aryldiazosulfonates corresponding to the formula 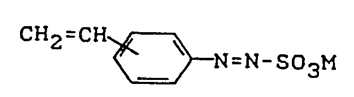 in which M is hydrogen, NH₄, NR'₄ (with R' = C₁₋₄ alkyl), Al, Ni, Zn, Cu, alkali metal or 1 equivalent alkaline earth metal.

7. A process for the production of the aryldiazosulfonates claimed in claims 1 to 6 by diazotization of arylamines containing polymerizable groups and subsequent reaction with sulfite.

8. The use of the aryldiazosulfonates claimed in claims 1 to 6 for the production of polymers.

9. Polymers containing recurring units bearing aryldiazosulfonate groups, these units containing
(i) at least one aromatic C₆₋₁₀ radical and
(ii) at least one diazosulfonate group attached to an aromatic radical.

10. Polymers as claimed in claim 9 in which the aromatic radicals (i) are phenylene and/or naphthylene.

11. Polymers as claimed in claim 9 or 10, in which the units bearing diazo sulfonate groups are derived from the aryldiazo sulfonates claimed in claims 1 to 6.

## Revendications

1. Aryldiazosulfonates comportant
(i) au moins un reste aromatique en C₆ à C₁₀,
(ii) au moins un groupe diazosulfonate lié à un reste aromatique et
(iii) au moins un groupe à non-saturation éthylénique polymérisable par voie radicalaire, lié à un reste aromatique en C₆ à C₁₀.

2. Aryldiazosulfonates suivant la revendication 1, dont les restes aromatiques (i) sont des restes phénylène et/ou naphtylène.

3. Aryldiazosulfonates suivant les revendications 1 et 2, dont les groupes non saturés (ii) comportent 1 ou 2 doubles liaisons C=C polymérisables.

4. Aryldiazosulfonates suivant les revendications 1 à 3, dont les groupes non saturés (iii) répondent à la formule
X-Rₙ- (I)
dans laquelle
X représente
-C=CH₂, -CCH₃=CH₂, -CH=CHCH₃, -CCN=CH₂, -CH=CHCN, -CCl=CH₂, -CH=CHCl, -OCOCH=CH₂, -OCOCH=CHCH₃, -OCOCCH₃=CH₂, -COOCCH₃=CH₂, -COOCH=CHCH₃, -CH=CH-CH=CH₂, -O-CH=CH₂, -O-CCH₃=CH₂ ou -O-CH=CH-CH₃,
R est un groupe alkylène en C₁ à C₁₀, arylène en C₆ à C₁₈, alkylène-oxy en C₁ à C₁₀, arylène-oxy en C₆ à C₁₈, (alkylène en C₁ à C₁₀)carboxyamino, (alkylène en C₁ à C₁₀)carbamoyle, arylènecarbamoyle en C₇ à C₁₉, alkylène-amino en C₁ à C₁₀ et arylène-amino en C₆ à C₁₈ et
n a la valeur 0 ou 1.

5. Aryldiazosulfonates suivant les revendications 1 à 4, dans lesquels les groupes diazosulfonates (II) sont liés aux mêmes restes aromatiques (i) que les groupes polymérisables (iii).

6. Aryldiazosulfonates de formule 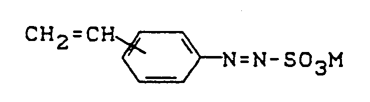 dans laquelle M est l'hydrogène, un groupe NH₄, NR'₄ (avec R' = alkyle en C₁ à C₄), Al, Ni, Zn, Cu, un métal alcalin ou 1 équivalent de métal alcalino-terreux.

7. Procédé de production d'aryldiazosulfonates suivant les revendications 1 à 6 par diazotation d'arylamines contenant des groupes polymérisables puis réaction avec un sulfite.

8. Utilisation des aryldiazosulfonates suivant les revendications 1 à 6 pour l'obtention de produits de polymérisation.

9. Polymères contenant des motifs récurrents porteurs de groupes aryldiazosulfonates, ces motifs contenant
(i) au moins un reste aromatique en C₆ à C₁₀ et
(ii) au moins un groupe diazosulfonate lié à un reste aromatique.

10. Polymères suivant la revendication 9, dans lequel les restes aromatiques (i) sont des restes phénylène et/ou naphtylène.

11. Polymères suivant les revendications 9 ou 10, dans lesquels les motifs contenant des groupes diazosulfonate sont dérivés d'aryldiazosulfonates suivant les revendications 1 à 6.
